# EUROPEAN PATENT SPECIFICATION

(11) **EP 0 880 490 B1**
(45) Date of publication and mention of the grant of the patent: **26.05.2004**
(21) Application number: 97946315.5
(22) Date of filing: 27.10.1997
(51) Int. Cl.: C07C 25/13, C07C 17/093

(54) **SYNTHESIS OF PERFLUORO AROMATIC BORANES AND BORATES**
SYNTHESE VON PERFLUORAROMATISCHEN BORANEN UND BORATEN
SYNTHESE DE BORANES ET DE BORATES AROMATIQUES PERFLUORO

(30) Priority: 25.10.1996 US 736655; 19.11.1996 US 751985; 21.11.1996 US 754622
(43) Date of publication of application: 02.12.1998
(73) Proprietor: BOULDER SCIENTIFIC COMPANY, Mead, CO 80542 (US)
(72) Inventor: ASKHAM, Fredric, Loveland, CO 80538 (US)
(74) Representative: Marchant, James Ian
(86) International application number: PCT/US1997/019384
(87) International publication number: WO 1998/017611

(56) References cited:
- US-A- 3 429 935
- US-A- 5 233 074

## Description

This application is a continuation-in-part of application Serial No. 08/736,655 filed October 25, 1996, application Serial No. 08/751,985 filed November 19, 1996 and application Serial No. 08/754,622 filed November 21, 1996.

### FIELD OF THE INVENTION

This invention relates to the preparation of certain fluoroaromatic compounds and to related boranes and borates.

More particularly, the invention relates to the preparation of bromopentafluorobenzene from hexafluorobenzene and to bromopentafluoro boranes and borates.

### BACKGROUND OF THE INVENTION

Various metallocene olefin polymerization catalysts comprise a pentafluorophenyl group frequently derived from expensive bromopentafluorobenzene. Accordingly, there is a need for a cost-effective method for converting readily available hexafluorobenzene to bromopentafluorobenzene.

Pentafluorobenzene magnesium halides (Grignard reagents) are known. Repress, et al. J. Organometallic Chem. (1969) 18:263-274 and Repress, et al., J. Organometallic Chem. (1969) 18:191-195 and United States patent 5,362,423. This synthesis is illustrated by Equation 1: 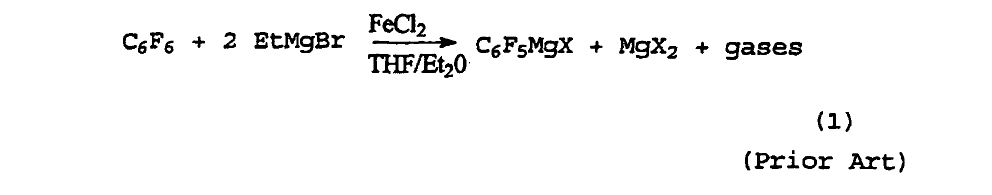 in which the three X's indicate two bromine atoms and one fluorine atom. The distribution of the bromine and fluorine atoms between the Grignard and the magnesium salt is not presently known.

### SUMMARY OF THE INVENTION

Pursuant to the invention bromopentafluorobenzene is synthesized by quenching a pentafluorobenzene magnesium halide with a halogenating agent, preferably bromine.

### DETAILED DESCRIPTION OF THE INVENTION

In a specific embodiment, this invention entails reaction of the C₆F₅MgX Grignard (see Equation 1) preferably in the vessel in which it is produced, with a halogenating agent, heat or in solution, in a non-interfering solvent to produce bromopentafluorobenzene. In generic embodiments of the invention, any Grignard reactant is used in the Equation 1 reaction which has the formula RMgY in which R is a hydrocarbyl radical containing a beta-hydrogen and Y is any halogen, i.e., fluorine, chlorine, bromine or iodine.

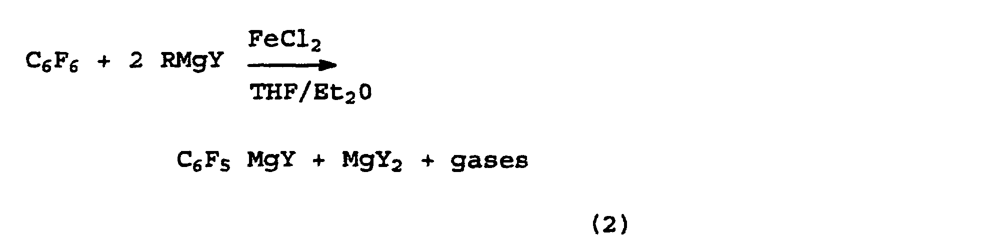

In the formula RMgY, Y is halogen, and preferably bromine or iodine and R is a C₁ to C₂₀, and preferably a C₂ to C₁₀, hydrocarbyl group such as, for example ethyl, propyl, butyl, pentyl, hexyl, heptyl, octyl, nonyl, decyl, including their branched chain isomers such as, for example, isopropyl, sec-butyl, tert-butyl, isobutyl, isopentyl, and cyclic alkyls such as for example, cyclohexyl and the like. The C₆F₅ MgY product is quenched, preferably in the Equation 2 reaction mixture, with a halogenating agent, heat or in solution in non-interfering, preferably a hydrocarbon or chlorinated hydrocarbon solvent to produce a first reaction mixture containing bromopentafluorobenzene. Useful halogenating agents include elemental bromine chlorine and iodine, carbon tetrachloride, carbon tetrabromide, chloroform, bromoform, and n-bromo or n-chloro succinamide. Elemental bromine is preferred.

The halogenating agent is added in an amount at least stoichiometric with respect to the pentafluorophenyl magnesium bromide. Preferably, the halogenating agent is added in an amount within ninety (90%) percent of the stoichiometric amount.

Suitable halogenating agent solvents are straight or branched chain, five to ten carbon atom, aliphatic hydrocarbons, benzene, toluene, and two to ten carbon atom halogenated aliphatic hydrocarbons. Methylene chloride is preferred.

### EXAMPLE 1

In a 2 l, N₂ purged flask were combined hexafluorobenzene (145 g), FeCl₂ (0.8 g) and THF (600 ml). The solution was cooled to 0-5°C, and ethyl magnesium bromide (3.12 M in Et₂O, 500 ml) was then added dropwise. After the addition was complete, the reaction mixture was stirred for one-half hour. A solution of bromine (125 g) in methylene chloride (250 ml) was then added dropwise to the cold (0-5°C) reaction mixture. After stirring 15 minutes, 4 N HCl was added until all solids were dissolved. The two phases were separated and the organics were washed sequentially with water, aqueous sodium bicarbonate and brine. After drying over sodium sulfate, the mixture was distilled providing 160 g of C₆F₅Br (83% yield).

The reaction mixture may also be reacted directly after removal of methylene chloride and otherwise without isolation of the C₆F₅Br with butyl lithium to produce C₆F₅Li which, in turn, is reacted with BCl₃ or BF₃ to produce (C₆F₅)₃Br.

## Claims

1. A method which comprises reacting C₆F₅MgY in which Y is F, Cl, Br, I with a halogenating agent to produce a halopentafluorobenzene which has the formula C₆F₅X in which X is a chlorine, bromine or iodine.

2. The claim 1 method in which the halogenating agent is bromine, chlorine, iodine, carbon tetrachloride, carbon tetrabromide, chloroform, bromoform, or an n-bromo or n-chloro succinamide.

3. A method according to claim 2 which comprises reacting C₆F₅MgY in which Y is F, Cl, Br, I with bromine to produce bromopentafluorobenzene.

4. A method for converting hexafluorobenzene (C₆F₆) to bromopentafluorobenzene (C₆F₅Br) which comprises:
(i) converting C₆F₆ to C₆F₅MgY in which Y is F, Cl, Br, I, and
(ii) reacting said C₆F₅MgY of step (i) with a halogenating agent in a non-interfering solvent, to provide a reaction mixture containing bromopentafluorobenzene in solution in said solvent.

5. The claim 4 method further comprising:
(iii) isolating said bromopentafluorobenzene from said step (ii) reaction mixture.

6. The claim 4 or claim 5 method in which said step (ii) non-interfering solvent is a 2 to 10 carbon atom hydrocarbon or a halogenated hydrocarbon.

7. A method which comprises
(i) reacting C₆F₆ with ethyl magnesium bromide in a medium comprising ethyl ether and tetrahydrofuran to produce C₆F₅MgBr in solution in said medium;
(ii) reacting said C₆F₅MgBr with bromine wherein C₆F₅Br is produced.

8. The claim 7 method further comprising
(iii) isolating said C₆F₅Br produced in step (ii).

9. The claim 8 method further comprising:
(iv) reacting said C₆F₅Br isolated in step (iii) with n-butyl lithium to convert said C₆F₅Br to C₆F₅Li.

## Patentansprüche

1. Verfahren, das Umsetzen von C₆F₅MgY, worin Y für F, Cl, Br, I steht, mit einem Halogenierungsmittel unter Herstellung eines Halogenpentafluorbenzols, das die Formel C₆F₅X, worin X für Chlor, Brom oder Jod steht, hat, umfasst.

2. Verfahren nach Anspruch 1, wobei das Halogenierungsmittel Brom, Chlor, Jod, Tetrachlorkohlenstoff, Tetrabromkohlenstoff, Chloroform, Bromoform oder ein n-Brom- oder n-Chlor-Succinamid ist.

3. Verfahren nach Anspruch 2, das das Umsetzen von C₆F₅MgY, worin Y für F, Cl, Br, I steht, mit Brom unter Herstellung von Brompentafluorbenzol umfasst.

4. Verfahren zur Umwandlung von Hexafluorbenzol (C₆F₆) in Brompentafluorbenzol (C₆F₅Br), das umfasst:
(i) Umwandeln von C₆F₆ in C₆F₅MgY, worin Y für F, Cl, Br, I steht, und
(ii) Umsetzen des C₆F₅MgY von Schritt (i) mit einem Halogenierungsmittel in einem nicht-wechselwirkenden Lösungsmittel unter Bereitstellung eines Reaktionsgemisches, das Brompentafluorbenzol in Lösung in dem Lösungsmittel enthält.

5. Verfahren nach Anspruch 4, das außerdem umfasst:
(iii) Isolieren des Brompentafluorbenzols aus dem Reaktionsgemisch von Schritt (ii).

6. Verfahren nach Anspruch 4 oder Anspruch 5, wobei das nicht-wechselwirkende Lösungsmittel von Schritt (ii) ein Kohlenwasserstoff mit 2 bis 10 Kohlenstoffatomen oder ein halogenierter Kohlenwasserstoff ist.

7. Verfahren, das umfasst
(i) Umsetzen von C₆F₆ mit Ethylmagnesiumbromid in einem Medium, das Ethylether und Tetrahydrofuran umfasst, um C₆F₅MgBr in Lösung in dem Medium herzustellen;
(ii) Umsetzen des C₆F₅MgBr mit Brom, wodurch C₆F₅Br hergestellt wird.

8. Verfahren nach Anspruch 7, das außerdem
(iii) Isolieren des C₆F₅Br, das in Schritt (ii) hergestellt wurde, umfasst.

9. Verfahren nach Anspruch 8, das außerdem
(iv) Umsetzen des C₆F₅Br, das in Schritt (iii) isoliert worden war, mit n-Butyl-lithium zur Umwandlung von C₆F₅Br in C₆F₅Li umfasst.

## Revendications

1. Procédé comprenant la réaction de C₆F₅MgY où Y est F, CI, Br, 1 avec un agent d'halogénation pour produire du halogénopentafluorobenzène de formule C₆F₅X où X est un atome de chlore, de brome ou d'iode.

2. Procédé selon la revendication 1 dans lequel l'agent d'halogénation est le brome, le chlore, l'iode, le tétrachlorure de carbone, le tétrabromure de carbone, le chloroforme, le bromoforme ou un N-bromo- ou un N-chloro-succinamide.

3. Procédé selon la revendication 2 qui comprend la réaction de CeFsMgY où Y est F, Cl, Br, I avec du brome pour produire du bromopentafluorobenzène.

4. Procédé pour convertir l'hexafluorobenzène (C₆F₆) en bromopentafluorobenzène (C₆F₅Br) comprenant :
(i) la conversion de C₆F₆ en C₆F₅MgY dans lequel Y est F, Cl, Br, I, et
(ii) la réaction dudit C₆F₅MgY de l'étape (i) avec un agent d'halogénation dans un solvant inerte, pour fournir un mélange de réaction contenant du bromopentafluorobenzène en solution dans ledit solvant.

5. Procédé selon la revendication 4 comprenant en outre :
(iii) l'isolement dudit bromopentafluorobenzène à partir du mélange de réaction de ladite étape (ii).

6. Procédé selon la revendication 4 ou la revendication 5 dans lequel le solvant inerte de ladite étape (ii) est un hydrocarbure de 2 à 10 atomes de carbone ou un hydrocarbure halogéné.

7. Procédé comprenant
(i) la réaction de C₆F₆ avec du bromure d'éthylmagnésium dans un milieu constitué d'éther éthylique et de tétrahydrofuranne pour produire du C₆F₅MgBr en solution dans ledit milieu ;
(ii) la réaction dudit C₆F₅MgBr avec du brome dans laquelle on produit du C₆F₅Br.

8. Procédé selon la revendication 7 comprenant en outre
(iii) l'isolement dudit C₆F₅Br produit dans l'étape (ii).

9. Procédé selon la revendication 8 comprenant en outre :
(iv) la réaction dudit C₆F₅Br que l'on a isolé dans l'étape (iii) avec du n-butyllithium pour convertir ledit C₆F₅Br en C₆F₅Li.
